# EUROPEAN PATENT SPECIFICATION

(11) **EP 3 463 108 B1**
(45) Date of publication and mention of the grant of the patent: **01.05.2024**
(21) Application number: 17807508.1
(22) Date of filing: 01.06.2017
(51) Int. Cl.: A61B 17/04, A61B 17/56, A61B 17/58, A61B 17/70, A61F 2/30, A61B 17/17, A61B 17/72, A61B 17/80

(54) **PLANTAR BONE FUSION PLATE**
PLANTARE KNOCHENFUSIONSPLATTE
PLAQUE DE FUSION D'OS PLANTAIRE

(30) Priority: 02.06.2016 US 201662344830 P
(43) Date of publication of application: 10.04.2019
(73) Proprietor: In2Bones USA, LLC, Memphis, TN 38119 (US); Varner, Kevin E., Memphis, TN 38119 (US); Heier, Keith A., Memphis, TN 38119 (US); Hanson, Travis W., Memphis, TN 38119 (US); Chambers, Casey M., Memphis, TN 38119 (US); Wahl, Rebecca, Hawkins, Escondido, CA 92025 (US)
(72) Inventor: VARNER, Kevin, E., Memphis, TN 38119 (US); HEIER, Keith, A., Memphis, TN 38119 (US); HANSON, Travis, W., Memphis, TN 38119 (US); CHAMBERS, Casey, M., Memphis, TN 38119 (US); WAHL, Rebecca, Hawkins, Escondido, CA 92025 (US)
(74) Representative: Dehns
(86) International application number: PCT/US2017/035527
(87) International publication number: WO 2017/210474

(56) References cited:
- WO-A1-2009/105201
- WO-A1-2013/055824
- US-A1- 2007 016 205
- US-A1- 2009 093 849
- US-A1- 2010 121 325
- US-A1- 2010 125 300
- US-A1- 2014 180 343
- US-B2- 6 890 335

## Description

### PRIORITY

This application claims the benefit of and priority to U.S. Patent Application No. 15/611,528 filed on June 1, 2017 and U.S. Provisional Application, entitled "Plantar Bone Fusion Plate," filed on June 2, 2016 and having application serial number 62/344,830.

### FIELD

The field of the present disclosure generally relates to securing bones together. More particularly, the field of the present disclosure relates to an apparatus and a method for fusing and compressing bones of the human body.

### BACKGROUND

A fusion bone plate implant may be utilized in conjunction with one or more fasteners so as to generate compression and stability at a bone interface. An implant coupled with fasteners generally serves to stabilize bones, or bone parts, relative to one another so as to promote bone fusion. In many applications, bone plates and fasteners are used to fuse bones, or bone parts, of the human body, such as bones in the foot, the ankle, the hand, the wrist, as well as various other portions of the body. Furthermore, during the course of certain medical procedures, a surgeon may immobilize one or more bones or the bone fragments by stabilizing the bones together in a configuration which approximates the natural anatomy. To this end, the surgeon may use fasteners to attach the bones to a bone plate implant so as to hold the bones in alignment with one another while they fuse together.

Bone plates are described in US 2010/125300.

### SUMMARY

A plantar bone plate as defined by claim 1 is provided for treating fractures of metatarsal bones.

In another embodiment, the two or more fixation apertures are configured to receive bone screws. In another embodiment, the two or more fixation apertures comprises at least four fixation apertures.

Also described is a method for a plantar bone plate for treating fractures of a metatarsal bone (not claimed) comprises providing a generally elongate member having an upper surface and a lower surface; disposing two or more fixation apertures along a longitudinal dimension of the elongate member; configuring the two or more fixation apertures to receive fasteners suitable for fastening the plantar bone plate to the metatarsal bone; forming a compression slot to receive a fastener perpendicular to the upper surface or at an oblique angle suitable for compressing adjacent portions of the metatarsal bone so as to encourage bone fusion; and applying a curvature along the elongate member such that the plantar bone plate mates with the anatomy of the plantar aspect of the metatarsal bone. Applying the curvature comprises forming at least a first bend and a second bend along the elongate member such that the plantar bone plate mates with the plantar anatomy of the 5^{th} metatarsal. Applying the curvature comprises forming a combination of one or more curves along the longitudinal dimension and a lateral dimension of the plantar bone plate.

### BRIEF DESCRIPTION OF THE DRAWINGS

The present invention provides a plantar bone plate for treating fractures of a metatarsal bone, as presented in claim 1. Preferred embodiments are set forth in the dependent claims .

The drawings refer to embodiments of the present disclosure in which:
Figure 1 illustrates an upper isometric view of an embodiment of a plantar bone plate for treating fractures of a metatarsal bone;
Figure 2 illustrates a lower isometric view of the embodiment of the plantar bone plate illustrated in Fig. 1;
Figure 3 illustrates a top plan view of the upper surface of the plantar bone plate illustrated in Fig. 1;
Figure 4 illustrates a side profile view of a longitudinally-directed curvature of the plantar bone plate illustrated in Fig. 1;
Figure 5 illustrates an exemplary use environment wherein the plantar bone plate of Fig. 1 is fastened onto a plantar aspect of a 5^{th} metatarsal bone across a fracture site;
Figure 6 illustrates a plan view of an exemplary use environment wherein the plantar bone plate of Fig. 1 has been implanted into a patient through a plantar lateral incision and fastened onto the 5^{th} metatarsal bone by way of two fasteners to treat a fracture; and
Figure 7 illustrates a profile view of the exemplary use environment shown in Fig. 6.

While the present disclosure is subject to various modifications and alternative forms, specific embodiments thereof have been shown by way of example in the drawings and will herein be described in detail.

### DETAILED DESCRIPTION

In the following description, numerous specific details are set forth in order to provide a thorough understanding of the present disclosure. It will be apparent, however, to one of ordinary skill in the art that the invention disclosed herein may be practiced without these specific details. In other instances, specific numeric references such as "first screw," may be made. However, the specific numeric reference should not be interpreted as a literal sequential order but rather interpreted that the "first screw" is different than a "second screw." Thus, the specific details set forth are merely exemplary. The specific details may be varied from and still be contemplated to be within the scope of the present disclosure. The term "coupled" is defined as meaning connected either directly to the component or indirectly to the component through another component. Further, as used herein, the terms "about," "approximately," or "substantially" for any numerical values or ranges indicate a suitable dimensional tolerance that allows the part or collection of components to function for its intended purpose as described herein.

In general, the present disclosure describes an apparatus and a method (not claimed)) for a plantar bone plate for treating Jones fractures, Pseudo Jones fractures, and Avulsion fractures of the metatarsal bones. The plantar bone plate comprises a generally elongate member having an upper surface and a lower, bone contact surface. Two or more fixation apertures are disposed along a longitudinal dimension of the elongate member. The fixation apertures are configured to receive fasteners suitable for fastening the plantar bone plate to the a metatarsal bone. Each of the fixation apertures comprises a countersunk surface disposed below the upper surface of the plantar bone plate. The countersunk surface is configured to allow a countersunk head of the fastener to assume a level that is flush with, or disposed below, the upper surface when the fastener is tightened to hold the plantar bone plate against the metatarsal bone. A compression slot is configured to receive a fastener that is oriented at an oblique angle for compressing adjacent portions of the metatarsal bone so as to encourage bone fusion. A curvature along the elongate member is configured to match the anatomy of the plantar aspect of the metatarsal bone to be fused. In one embodiment, the curvature is comprised of at least a first bend and a second bend that are configured to mate the plant bone fusion plate with the anatomy of the plantar aspect of the 5^{th} metatarsal bone.

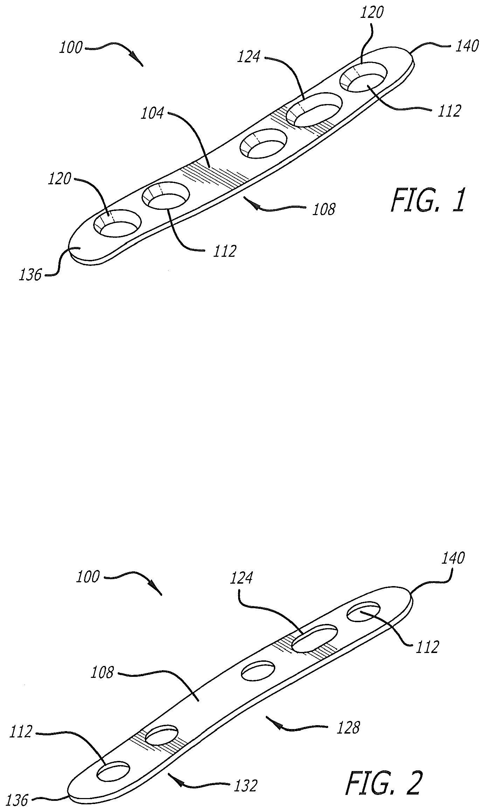

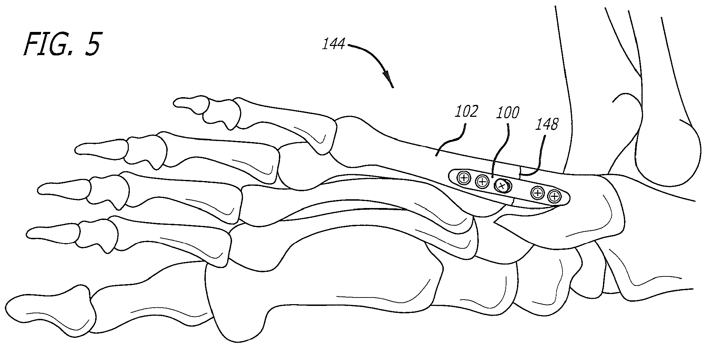

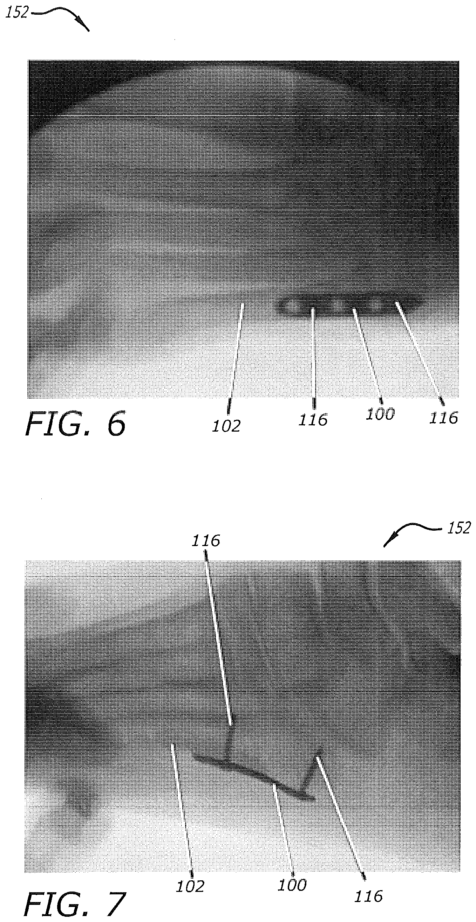

Figures 1-5 illustrate an embodiment of a plantar bone plate 100 configured to treat Jones fractures, Pseudo Jones fractures, and Avulsion fractures of a 5^{th} metatarsal 102. Although the plantar bone plate 100 is shown in Fig. 5 to be fusing the 5^{th} metatarsal, it should be understood that the plantar bone plate may be adapted for treating fractures of any of the metatarsal bones, without limitation. The plantar bone plate 100 comprises a generally elongate member having an upper surface 104 and a lower, bone contact surface 108. Disposed along the plantar bone plate 100 are two or more fixation apertures 112 suitable for receiving fasteners 116, such as bone screws as shown in Figs. 6-7. Preferably, the plantar bone plate 100 includes 4-5 fixation apertures 112, although in other embodiments the plantar bone plate may include less than 4 or more than 5 fixation apertures 112. The plantar bone plate 100 preferably is comprised of a semi-rigid material, such as a biocompatible metal or PEEK, possessing a tensile strength suitable for immobilizing adjacent bone portions of the 5^{th} metatarsal 102.

The fixation apertures 112 are configured to each receive a fastener 116, such as a bone screw that may be utilized to fasten the plantar bone plate 100 onto the 5^{th} metatarsal 102, as disclosed above and shown in Figs. 5-7. Each fixation aperture 112 comprises a countersunk surface 120 disposed within the fixation aperture and below the upper surface 104 of the bone plate. As will be appreciated, the countersunk surface 120 allows a countersunk head of the fastener 116 to assume a level that is either above, flush with, or disposed below the upper surface 104 when the fastener is tightened to hold the plantar bone plate 100 against the bone. It is envisioned that the countersunk surfaces 120 may be implemented with any of various suitable chamfer angles, including, but not limited to, 60°, 82°, 90°, 100°, 110°, or 120°. In some embodiments, however, the chamfer angle of the countersunk surfaces 120 preferably is either 82° or 90°. Further, it is contemplated that the countersunk surfaces 120 comprise a locking feature that operates to prevent the fasteners 116 from backing out of the bone after having been implanted in a patient.

The plantar bone plate 100 preferably comprises at least one compression slot 124 configured to receive a fastener 116. Similar to the fixation apertures 112, the compression slot 124 may receive the fastener 116 at an angle of substantially 90° with respect to the plane of the plantar bone plate 100. Unlike the fixation apertures, however, the compression slot 124 may alternatively receive the fastener 116 at an oblique angle with respect to the plane of the plantar bone plate 100. The oblique angle of the fastener 116 facilitates compressing adjacent bone portions together so as to encourage bone fusion. The fastener 116 may be any component of hardware having a head configured to abut the surface of plantar bone plate 100 and a shaft configured to secure bone portions together in a fixed configuration. In some embodiments, the fastener 116 may comprise a lag screw which includes a head that is rounded, or tapered, and coupled to a shaft, or a shank, that has an unthreaded portion adjacent to the head and a threaded portion that ends at a tip.

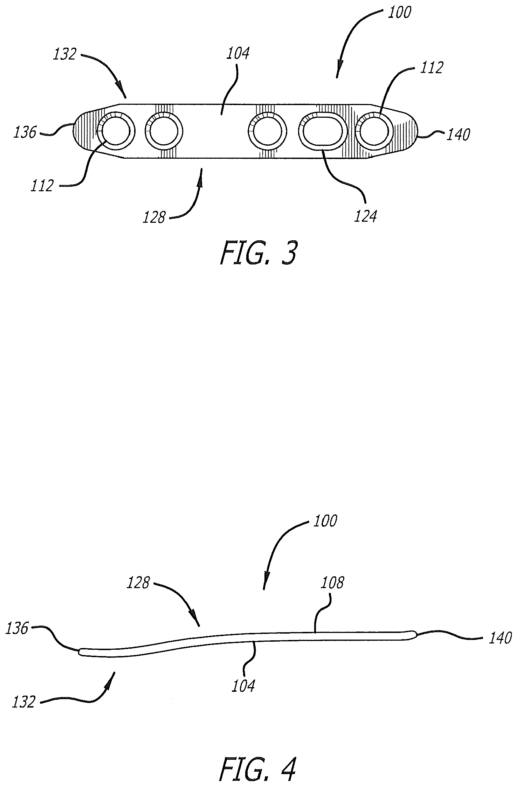

As best illustrated in Fig. 4, a longitudinal dimension of the plantar bone plate 100 is comprised of a first bend 128 and a second bend 132. The first bend 128 is comprised of a smooth, slightly curved portion that is concaved toward the upper surface 104 and extending from substantially a middle portion of the plantar bone plate 100 to a beginning of the second bend 132. The second bend 132 is comprised of a curved portion concaved away from the upper surface 104 and extending from the first bend 128 to a proximal end 136 of the plantar bone plate 100. A relatively flat portion of the plantar bone plate 100 extends from the first bend 128 to a distal end 140 of the bone plate. In some embodiments, however, one or more curved portions may be incorporated into the portion of the plantar bone plate 100 between the first bend 128 and the distal end 140.

As shown in Fig. 4, the first bend 128 and the second bend 132 comprise a tangent reverse curve, wherein the second bend 132 comprises a relatively smaller radius than a radius of the first bend 128. As will be appreciated, the radius and concavity of the first bend 128, and the radius and concavity of the second bend 132 are selected such that the curvature of the plantar bone plate 100 substantially matches, or mates with, the plantar anatomy of the 5^{th} metatarsal 102, as shown in Fig. 5. It is contemplated, however, that the radii and concavities of the first and second bends 128, 132 may be varied from those illustrated and disclosed herein, without limitation, so as to mate the plantar bone plate 100 to the anatomy of any of the metatarsal bones, as needed.

Moreover, it should be recognized that the plantar bone plate 100 is not to be limited to bends along the longitudinal dimension, but rather the plantar bone plate 100 may comprise a curvature along a lateral dimension of the plantar bone plate 100 that is substantially perpendicular to the longitudinal dimension, as well as a combination of curvatures along the longitudinal and lateral dimensions of the plantar bone plate. In some embodiments, the curvature may change along the longitudinal and lateral dimensions as a function of distance from a middle, or other reference location, of the plantar bone plate 100. In some embodiments, the curvatures along the longitudinal and lateral dimensions may be selected to match a specific anatomy of a bone to be treated other than the metatarsals. Accordingly, it should be understood that the plantar bone plate 100 may be implemented with any combination of topological features without deviating from the scope of the present disclosure.

Figure 5 illustrates an exemplary use environment 144 wherein the plantar bone plate 100 is fastened onto the plantar aspect of the 5^{th} metatarsal 102 across a bone fracture 148. It is contemplated that in practice, the plantar bone plate 100 may be implanted into the patient through a plantar lateral incision. For example, in an exemplary environment 152 illustrated in Figs. 6-7, the plantar bone plate 100 has been implanted into a patient through a plantar lateral incision and fastened onto the 5^{th} metatarsal 102 by way of two fasteners 116 to treat a fracture 148. As will be appreciated, with the plantar bone plate 100 fastened to the plantar aspect of the 5^{th} metatarsal, the plantar bone plate is predominantly placed in tension under normal gait cycle loading, thereby naturally maintaining compression at the bone fracture 148. As such, it should be recognized that implanting the plantar bone plate 100 onto the plantar aspect of the 5^{th} metatarsal is mechanically superior to placing the bone plate on a dorsal aspect of the bone. Further, it should be understood that the plantar bone plate 100 is not to be limited to treating fractures of the 5^{th} metatarsal 102. Rather, it is contemplated that the plantar bone plate 100 may be adapted for treating various types of fractures to any of the metatarsal bones, as well as other bones of the human body, as needed.

It is envisioned that the embodiments discussed herein may be coupled with various surgical instruments that are configured for implanting the plantar bone plate 100 and fasteners 116 into patients. In some exemples, the surgical instruments may include, without limitation, plate trials, wires, drills, drill guides, depth gages, cup and cone reamers, screw drivers, plate benders, and the like. It is further envisioned that the plantar bone plate 100, accompanying fasteners 116, and the selected surgical instruments are to be suitably sterilized for surgeries and packaged into sterilized containers. In some exemples, the plantar bone plate 100 may be packaged into a first sterile container, the fasteners 116 may be packaged into a second sterile container, and the instruments may be packaged into a third sterile container. The first, second, and third sterile containers may then be bundled together into a single, exterior container, thereby forming a convenient surgery-specific bone fusion implant package. It is envisioned that other packaging techniques will be apparent to those skilled in the art without deviating from the scope of the invention as defined by the claims.

While the invention has been described in terms of particular variations and illustrative figures, those of ordinary skill in the art will recognize that the invention is not limited to the variations or figures described. In addition, where methods and steps described above indicate certain events occurring in certain order, those of ordinary skill in the art will recognize that the ordering of certain steps may be modified and that such modifications are in accordance with the variations of the invention. Additionally, certain of the steps may be performed concurrently in a parallel process when possible, as well as performed sequentially as described above. The present disclosure is to be understood as not limited by the specific embodiments described herein, but only by scope of the appended claims.

## Claims

1. A plantar bone plate for treating fractures of a metatarsal bone, comprising:
a generally elongate member having an upper surface (104) and a lower, bone contact surface (108) the elongate member comprised of a semi-rigid material;
two or more fixation apertures (112) disposed along a longitudinal dimension of the elongate member and configured to receive fasteners (116) suitable for fastening the plantar bone plate to the metatarsal bone wherein the plantar bone plate is comprised of a material possessing a tensile strength suitable for immobilizing adjacent bone portions of the metatarsal bone;
a compression slot (124) configured to receive a fastener perpendicular to or at an oblique angle with respect to the upper surface for compressing adjacent portions of the metatarsal bone so as to encourage bone fusion; and **characterised by**
a curvature along the elongate member configured to mate with the anatomy of the plantar aspect of the metatarsal bone wherein the curvature is comprised of a first bend (128) and a second bend (132) along the longitudinal dimension of the plantar bone plate, the first bend and the second bend comprising a tangent reverse curve, wherein the radius and concavity of the first bend and the radius and concavity of the second bend are configured to mate with the anatomy of the plantar aspect of the metatarsal bone comprising a 5^{th} metatarsal, and wherein the second bend comprises a smaller radius than a radius of the first bend; wherein the two or more fixation apertures (112) each is comprised of a countersunk surface (120) disposed below the upper surface of the bone plate and configured to allow a countersunk head of a fastener to assume a level that is above, flush with, or disposed below the upper surface when the fastener is tightened to hold the plantar bone plate against the metatarsal bone; and wherein the countersunk surface comprises a chamfer angle that ranges between 60° and 120°, and a locking feature; and wherein the countersunk surface is configured to prevent the fastener from backing out after being implanted into a bone.

2. The bone plate of claim 1, wherein the two or more fixation apertures (112) are configured to receive bone screws.

3. The bone plate of any preceding claim, wherein the two or more fixation apertures (112) comprises at least four fixation apertures.

## Patentansprüche

1. Plantare Knochenplatte zur Behandlung von Frakturen eines Mittelfußknochens, umfassend:
ein allgemein längliches Element, das eine obere Fläche (104) und eine untere Knochenkontaktfläche (108) aufweist, wobei das längliche Element aus einem halbstarren Material besteht;
zwei oder mehr Fixierungsöffnungen (112), die entlang einer Längsabmessung des länglichen Elements angeordnet und so konfiguriert sind, dass sie Befestigungselemente (116) aufnehmen, die zum Befestigen der plantaren Knochenplatte am Mittelfußknochen geeignet sind, wobei die plantare Knochenplatte aus einem Material besteht, das eine Zugfestigkeit besitzt, die zum Immobilisieren aneinandergrenzender Knochenabschnitte des Mittelfußknochens geeignet ist;
einen Kompressionsschlitz (124), der so konfiguriert ist, dass er ein Befestigungselement senkrecht oder in einem schrägen Winkel in Bezug auf die obere Fläche aufnimmt, um aneinandergrenzende Abschnitte des Mittelfußknochens zu komprimieren, um die Knochenfusion zu fördern; und **gekennzeichnet durch**
eine Krümmung entlang des länglichen Elements, die so konfiguriert ist, dass sie mit der Anatomie der plantaren Seite des Mittelfußknochens zusammenpasst, wobei die Krümmung aus einer ersten Biegung (128) und einer zweiten Biegung (132) entlang der Längsabmessung der plantaren Knochenplatte besteht, wobei die erste Biegung und die zweite Biegung eine tangentiale Rückwärtskurve umfassen, wobei der Radius und die Konkavität der ersten Biegung und der Radius und die Konkavität der zweiten Biegung so konfiguriert sind, dass sie mit der Anatomie der plantaren Seite des Mittelfußknochens, die einen 5-ten Mittelfußknochen umfasst, zusammenpassen, und wobei die zweite Biegung einen kleineren Radius umfasst als einen Radius der ersten Biegung; wobei die zwei oder mehr Fixierungsöffnungen (112) jeweils aus einer Senkfläche (120) bestehen, die unter der oberen Fläche der Knochenplatte angeordnet und so konfiguriert ist, dass sie es einem Senkkopf eines Befestigungselements ermöglicht, eine Ebene einzunehmen, die über, bündig mit, oder unter der oberen Fläche angeordnet ist, wenn das Befestigungselement festgezogen ist, um die plantare Knochenplatte am Mittelfußknochen zu halten; und wobei die Senkfläche einen Fasenwinkel im Bereich zwischen 60° und 120° und ein Verriegelungsmerkmal aufweist; und wobei die Senkfläche so konfiguriert ist, dass sie verhindert, dass sich das Befestigungselement, nachdem es in einen Knochen implantiert wurde, herauslöst.

2. Knochenplatte nach Anspruch 1, wobei die zwei oder mehr Fixierungsöffnungen (112) so konfiguriert sind, dass sie Knochenschrauben aufnehmen.

3. Knochenplatte nach einem vorstehenden Anspruch, wobei die zwei oder mehr Fixierungsöffnungen (112) mindestens vier Fixierungsöffnungen umfassen.

## Revendications

1. Plaque osseuse plantaire pour traiter des fractures d'un os métatarsien, comprenant :
un élément généralement allongé présentant une surface supérieure (104) et une surface de contact osseux inférieure (108), l'élément allongé étant constitué d'un matériau semi-rigide ;
au moins deux ouvertures de fixation (112) disposées le long d'une dimension longitudinale de l'élément allongé et configurées pour recevoir des fixations (116) appropriées pour fixer la plaque osseuse plantaire à l'os métatarsien, dans laquelle la plaque osseuse plantaire est constituée d'un matériau possédant une résistance à la traction adapté pour immobiliser des parties d'os adjacentes de l'os métatarsien ;
une fente de compression (124) configurée pour recevoir un élément de fixation perpendiculaire ou à un angle oblique par rapport à la surface supérieure pour comprimer des parties adjacentes de l'os métatarsien de manière à favoriser une fusion osseuse ; et **caractérisé par**
une courbure le long de l'élément allongé configurée pour s'apparier à l'anatomie de l'aspect plantaire de l'os métatarsien, dans laquelle la courbure est constituée d'un premier coude (128) et d'un second coude (132) le long de la dimension longitudinale de la plaque osseuse plantaire, le premier coude et le second coude comprenant une courbe inverse tangente, dans laquelle le rayon et la concavité du premier coude et le rayon et la concavité du second coude sont configurés pour s'apparier à l'anatomie de l'aspect plantaire de l'os métatarsien comprenant un 5^{e} métatarsien, et dans laquelle le second coude comprend un rayon plus petit qu'un rayon du premier coude ; dans laquelle les deux ouvertures de fixation (112) ou plus comprennent chacune une surface fraisée (120) disposée au-dessous de la surface supérieure de la plaque osseuse et configurée pour permettre à une tête fraisée d'un élément de fixation de prendre un niveau qui est au-dessus, au ras de, ou disposé sous la surface supérieure lorsque la fixation est serrée pour maintenir la plaque osseuse plantaire contre l'os métatarsien ; et dans laquelle la surface fraisée comprend un angle de chanfrein compris entre 60° et 120°, et un élément de verrouillage ; et dans laquelle la surface fraisée est configurée pour empêcher la fixation de reculer après avoir été implantée dans un os.

2. Plaque osseuse selon la revendication 1, dans laquelle les deux ouvertures de fixation (112) ou plus sont configurées pour recevoir des vis à os.

3. Plaque osseuse selon une quelconque revendication précédente, dans laquelle les deux ouvertures de fixation (112) ou plus comprennent au moins quatre ouvertures de fixation.
